# EUROPÄISCHE PATENTSCHRIFT

(11) **EP 3 663 806 B1**
(45) Veröffentlichungstag und Bekanntmachung des Hinweises auf die Patenterteilung: **11.02.2026**
(21) Anmeldenummer: 18209798.0
(22) Anmeldetag: 03.12.2018
(51) Int. Cl.: G01T 1/24, H01L 25/00

(54) **PHOTONENDETEKTOR, VERFAHREN ZUR HERSTELLUNG EINES PHOTONENDETEKTORS UND RÖNTGENGERÄT**
PHOTON DETECTOR, METHOD FOR PRODUCING A PHOTON DETECTOR AND X-RAY DEVICE
DÉTECTEUR DE PHOTONS, PROCÉDÉ DE FABRICATION D'UN DÉTECTEUR DE PHOTONS ET APPAREIL À RAYONS X

(43) Veröffentlichungstag der Anmeldung: 10.06.2020
(73) Patentinhaber: Siemens Healthineers AG, 91301 Forchheim (DE)
(72) Erfinder: ERGLER, Thorsten, 91054 Erlangen (DE); HOSEMANN, Michael, 91056 Erlangen (DE)
(74) Vertreter: Siemens Healthineers Patent Attorneys

(56) Entgegenhaltungen:
- WO-A2-2022/122374
- CN-A- 105 556 673
- DE-A1- 102007 022 197
- DE-A1- 102016 201 808
- DE-A1- 102016 221 481

## Beschreibung

Die Erfindung betrifft einen Photonendetektor, insbesondere einen Röntgenstrahlungsdetektor. Des Weiteren betrifft die Erfindung ein Verfahren zur Herstellung eines solchen Photonendetektors sowie ein medizinisches Röntgengerät mit einem solchen Photonendetektor.

Photonendetektoren kommen neben herkömmlichen Kameras (Fotoapparaten oder Bewegt-Bild-Kameras) regelmäßig in (vorzugsweise medizinischen) Röntgen-Bildgebungsgeräten zum Einsatz. Herkömmliche Photonendetektoren weisen dabei eine für Röntgenstrahlung sensitive Schicht aus einem Szintillator-Material (meist eine Keramik) auf, in der einfallende Röntgenstrahlung in Strahlung im sichtbaren Spektralbereich gewandelt wird. Diese sichtbare Strahlung wird rückseitig zu einfallenden Röntgenstrahlung mittels Fotodioden, CMOS-Sensoren oder dergleichen erfasst und zur Generierung des Röntgenbilds ausgewertet. Die Erfassung der sichtbaren Strahlung erfolgt dabei in vergleichsweise langen Zeitintervallen, während derer die einfallende Röntgenstrahlung quasi aufintegriert wird.

Um insbesondere für Patienten die Röntgenstrahlungs-Dosis zu verringern, werden sogenannte quanten- oder photonenzählende Photonendetektoren entwickelt oder sogar bereits eingesetzt. Bei diesem kommt als für (Röntgen-) Strahlung sensitive Schicht (als "Sensorelement" bezeichnet) ein Halbleitermaterial, beispielsweise Cadmium-Tellurid (kurz: CdTe) zum Einsatz. In diesem erzeugt die einfallende (Röntgen-) Strahlung, konkret jedes einfallende (Röntgen-)Photon eine Anzahl von Ladungsträgern (insbesondere mehrere Elektronen-Loch-Paare), der aufgrund einer an das Sensorelement angelegten (Hoch-) Spannung zu einer Flachseite des Sensorelements driftet und dort mittels einer mit dem Sensorelement kontaktierten Auswerte-Schaltung erfasst wird.

Um eine Ortsauflösung der detektierten Ereignisse zu ermöglichen, ist das Sensorelement in mehrere rasterartig angeordnete Bildpunkte (im Folgenden als "Sensorpixel" bezeichnet) unterteilt. Die Auswerte-Schaltung weist dabei jeweils einen einem jeden Sensorpixel zur Detektion zugeordneten "Detektorpixel" auf, der mit dem jeweiligen Sensorpixel signalübertragungstechnisch kontaktiert ist. Die Auswerte-Schaltung ist dabei üblicherweise als integrierter Schaltkreis, konkret als anwendungsspezifischer Schaltkreis (ASIC) ausgebildet.

Um ein möglichst geringes Rauschen und eine hohe Bildqualität zu ermöglichen, müssen diese integrierten Schaltkreise mit möglichst geringem Abstand an die Sensorpixel angekoppelt werden. Üblicherweise ist jedoch die von dem Sensorelement und dessen einzelnen Sensorpixeln aufgespannte Fläche größer als die durch moderne Fertigungstechnologien zur Auswertung dieser Sensorpixel benötigte ASIC-Fläche. Um jedoch den möglichst geringen Abstand zu den Sensorpixeln zu ermöglichen, wird die Geometrie der Detektorpixel und somit des jeweiligen ASIC an die Geometrie des Sensorelements und somit der durch die Sensorpixel gebildeten Matrix angeglichen. Dies führt wiederum zu einer Kostensteigerung für die ASICs. Außerdem müssen, um den jeweiligen ASIC mit einer nachgeordneten Leitungsebene, Stromversorgung oder dergleichen kontaktieren zu können, aufwendige und somit kostenintensive Durchkontaktierungen (sogenannte "Through Silicon Vias", "TSVs") in den ASIC eingebracht werden.

Aus der Druckschrift DE 10 2014 213 734 A1 ist eine bildgebende Vorrichtung für elektromagnetische Strahlung, insbesondere für Röntgen- und/oder Gamma-Strahlung, bekannt, welche eine Schichtung aus einer Anzahl von Detektionselementen, einer Anzahl von Auslese-Platinen, und einer Basisplatine umfasst, wobei das oder jedes Detektionselement mit jeweils einer Auslese-Platine über eine Mehrzahl von ersten Löt-Kontaktierungen elektrisch kontaktiert ist, wobei die oder jede Auslese-Platine eine Mehrzahl von Durchkontaktierungen aufweist, und wobei die oder jede Auslese-Platine mit der Basisplatine über eine Mehrzahl von zweiten Löt-Kontaktierungen elektrisch kontaktiert ist.

Aus der Druckschrift DE 10 2014 221 829 A1 ist ein Verfahren zur Herstellung eines Sensorboards für ein Detektormodul bekannt, wobei eine Mehrzahl von Ausleseeinheiten bereitgestellt wird, wobei die Ausleseeinheiten in einem Stapelaufbau jeweils auf einer gemeinsamen Sensorschicht positioniert werden, und wobei nach erfolgter Positionierung aller Ausleseeinheiten diese unter Ausbildung eines Hybrids gemeinsam an der Sensorschicht fixiert werden.

Aus der DE 10 2016 221 481 A1 ist ein Strahlungsdetektor mit einer Zwischenschicht offenbart, die zwischen einer Detektionsschicht mit einer Anzahl von Detektionselementen und einer Anzahl von Ausleseeinheiten angeordnet ist. Dabei weist die Zwischenschicht eine Vielzahl elektrisch leitendender Verbindungen zwischen den Detektionselementen und den Ausleseeinheiten auf.

Die Druckschrift CN 105 556 673 A offenbart einen Detektor mit elektronischen Schaltungen in einer Vergussmasse , wobei auf der Vergussmasse ein Isolierelement mit elektrischen Leitungen ausgebildet ist.

Die Druckschrift DE 10 2007 022197 A1 offenbart einen Strahlungsdetektor mit einer Zwischenschicht, die zwischen einer Detektionsschicht mit einer Anzahl von Detektionselementen und einer Anzahl von Ausleseeinheiten angeordnet ist, wobei die Zwischenschicht eine Vielzahl elektrisch leitendender Verbindungen zwischen den Detektionselementen und den Ausleseeinheiten aufweist.

Diese Aufgabe wird erfindungsgemäß gelöst durch einen Photonendetektor mit den Merkmalen des Anspruchs 1. Des Weiteren wird diese Aufgabe erfindungsgemäß gelöst durch ein Verfahren zur Herstellung eines Photonendetektors mit den Merkmalen des Anspruchs 7. Außerdem wird diese Aufgabe erfindungsgemäß gelöst durch ein Röntgengerät mit den Merkmalen des Anspruchs 9. Weitere vorteilhafte und teils für sich erfinderische Ausführungsformen und Weiterentwicklungen der Erfindung sind in den Unteransprüchen und der nachfolgenden Beschreibung dargelegt.

Der erfindungsgemäße Photonendetektor bildet vorzugsweise einen Röntgenstrahlungsdetektor. Dazu weist der Photonendetektor wenigstens ein Sensorelement auf, das durch ein Halbleitermaterial gebildet ist und das für einfallende Strahlung - vorzugsweise Röntgenstrahlung - sensitiv ist. Außerdem bildet dieses Sensorelement eine Pixelmatrix (auch als "Sensormatrix" bezeichnet) mit - konkret aus - einer Anzahl von Sensorpixeln. Der Photonendetektor weist außerdem (insbesondere wenigstens) eine Detektorschaltung auf, die dem Sensorelement in Strahlungseinfallsrichtung nachgelagert ist und die zur Erfassung von in dem Halbleitermaterial des Sensorelements strahlungsbedingt generierten Ladungsträgern dient. Die Detektorschaltung umfasst dabei einen integrierten Schaltkreis (nachfolgend kurz als "ASIC" bezeichnet), der wiederum eine insbesondere zumindest zu einer Teilmenge der Sensorpixel korrespondierende Anzahl an Detektorpixeln aufweist. Diese Detektorpixel sind dabei mit den Sensorpixeln (zumindest der Teilmenge) signalübertragungstechnisch kontaktiert. Eine Flächenausdehnung (d. h. zumindest eine Kantenlänge) des ASIC unterschreitet dabei jedoch die Flächenausdehnung (d. h. die korrespondierende Kantenlänge) des Sensorelements um mehr als eine Pixelbreite der Sensorpixel - vorzugsweise um mehr als zwei, drei oder vier Pixelbreiten. Die Detektorschaltung weist dabei ein den ASIC umgebendes Gehäuse auf, in das der ASIC eingebettet ist. Auf einer dem Sensorelement zugewandten Kontaktseite (im Folgenden als "Pixelseite" bezeichnet) des Gehäuses (sowie vorzugsweise auch auf dem ASIC selbst) ist eine Umkontaktierungslage ausgebildet, vorzugsweise stoffschlüssig appliziert. In dieser Umkontaktierungslage sind Kontaktstellen zur signalübertragungstechnischen (insbesondere galvanischen) Verbindung der Detektorpixel mit den entsprechend zugeordneten Sensorpixeln sowie Leiterbahnen zur Verbindung dieser Kontaktstellen mit den Detektorpixeln des ASICs ausgebildet. Diese Umkontaktierungslage dient zur flächigen Aufspreizung der dem Sensorelement zugewandten, einem jeden Detektorpixel zugeordnete Kontaktstellen tragende Kontaktfläche des ASIC auf eine (insbesondere rückseitige) diesem ASIC zugewandte Gegenkontaktfläche des Sensorelements.

Durch die Nutzung des Gehäuses und der darauf angeordneten Umkontaktierungslage kann mithin ein im Vergleich zum Sensorelement, zumindest zur Gegenkontaktfläche kleinerer ASIC als Detektorschaltung eingesetzt werden. Dadurch können vorteilhafterweise (zumindest materialbedingte) Herstellungskosten für vergleichsweise großflächige ASICs vermieden werden. Außerdem kann Bauraum für Kontaktierungen des jeweiligen ASICs mit in Strahlungseinfallsrichtung dem ASIC nachgeordneten Signalführungsmitteln, einer übergeordneten Steuer- und Auswerteeinheit und/oder einer Stromversorgungseinheit freigehalten werden, sodass kostenintensive Durchkontaktierungen durch den ASIC selbst (insbesondere in Form von TSVs) entfallen können. Außerdem kann die durch das ASIC und das Gehäuse gebildete Detektorschaltung auch insbesondere direkt - vorzugsweise mittels der Umkontaktierungslage - an das Sensorelement signalübertragungstechnisch angebunden werden. Eine zusätzliche (bspw. löttechnische) Anbindung des ASICs an ein zwischengelagertes, separates Kontaktierungselement (bspw. einen sogenannten Interposer) kann somit vorteilhafterweise entfallen. Dadurch kann eine besonders hohe Fertigungspräzision insbesondere durch den Wegfall zusätzlicher Signalverbindungen (bspw. Lötverbindungen mit dem separaten Kontaktierungselement) erreicht werden. Des Weiteren wird auch die Positionierung der jeweiligen Detektorschaltung zum korrespondierenden Sensorelement vereinfacht, da diese unmittelbar aneinander angebunden werden.

In einer bevorzugten Ausführung ist der ASIC oberflächenbündig und an seinen (quer zur Pixelseite verlaufenden) Kanten spaltfrei in das Gehäuse - das vorzugsweise durch ein Gießharz gebildet ist -eingebettet. Das heißt, dass das Gehäuse den ASIC kantenseitig - gegebenenfalls auch auf der der Pixelseite abgewandten Rückseite - umgibt.

Grundsätzlich sind zur signalübertragungstechnischen Anbindung der Detektorschaltung an das Sensorelement, konkret also der den Detektorpixeln zugeordneten Kontaktstellen an die entsprechenden Sensorpixel, verschiedene Verbindungsverfahren zum Erstellen einer elektrisch leifähigen Verbindung (bspw. Löten, Leitkleben und dergleichen) denkbar. Im Fall einer Lötverbindung kommen aber bevorzugt bleifreie Niedertemperaturlote zum Einsatz, um eine thermische Schädigung des Gehäuses zu vermeiden.

Erfindungsgemäß ist die Umkontaktierungslage durch ein Dünnschicht-Applikationsverfahren ausgebildet. Beispielsweise wird die Umkontaktierungslage unter Nutzung eines Dampfphasen-Abscheidungsverfahrens (bspw. zumindest zur Ausbildung von Metallisierungskeimen), z. B. PVD ("physical vapor depositioning"), eines Maskenbelichtungsverfahrens oder dergleichen oder einer Kombination daraus, ausgebildet.

In einer bevorzugten Ausführung ist die Detektorschaltung durch den ASIC mittels eines als "Fan-Out Wafer-Level Chip-Scale Packaging" bezeichneten Verfahrens gefertigt. Die Detektorschaltung bildet diesem Fall mithin ein Bauteil, das den ASIC, das diesen umgebende Gehäuse und die wenigstens eine Umkontaktierungslage umfasst. Derartige Detektorschaltungen bzw. Bauteile können als vorgefertigte Elemente zugekauft oder gegebenenfalls auch nutzerspezifisch hergestellt werden. Vorteilhafterweise können dadurch im Vergleich zu den vorstehend beschriebenen vergleichsweise großflächigen ASICs, die an die Fläche des Sensorelements angepasst sind, Herstellungskosten eingespart werden. Außerdem wird es möglich, auch für verschiedene Photonendetektoren, die Sensorelemente mit unterschiedlicher Geometrie aufweisen, stets den gleichen ASIC bei lediglich an das entsprechende Sensorelement angepasstem Gehäuse und entsprechend auch angepasster Umkontaktierungslage einzusetzen. Dies ermöglicht mithin den Einsatz von Gleichteilen über verschiedene Photonendetektoren hinweg, was wiederum zu einer Kostenersparnis beiträgt.

In einer besonders zweckmäßigen Ausführung weist die Detektorschaltung im Bereich des Gehäuses wenigstens eine Durchkontaktierung auf, die sich vorzugsweise von der Pixelseite zur dieser abgewandten Rückseite oder "Anschlussseite" der Detektorschaltung erstreckt. Diese Durchkontaktierung dient dabei zur signalübertragungstechnischen Anbindung des ASICs an eine nachgelagerte Steuer- und Auswerteeinheit und/oder nachgelagerte Signalführungsmittel oder dergleichen. D. h., dass der ASIC der jeweiligen Detektorschaltung nicht selbst mit einer oder mehreren Durchkontaktierungen (insbesondere einem TSV) versehen ist. Dadurch können weiter Herstellungskosten für die Detektorschaltung eingespart werden, da aufwändige Durchkontaktierungen, konkret als TSV bezeichnet durch den ASIC hindurch entfallen können. Die Durchkontaktierungen durch das vorzugsweise aus Kunststoff gebildete Gehäuse lassen sich hingegen mit vergleichsweise einfachen und kostengünstigen Fertigungsverfahren - beispielsweise Laserstrahlbohren, Urformen oder dergleichen herstellen. Des Weiteren ist die oder die jeweilige Durchkontaktierung mittels der oder einer weiteren Umkontaktierungslage an den ASIC angebunden. Durch die oder die jeweilige Durchkontaktierung ist es außerdem möglich, Leitstrukturen, beispielsweise Leiterbahnen, Drähte oder dergleichen, die um die Außenkanten des ASICs oder des Gehäuses selbst von der Pixelseite zur Rückseite geführt werden, zu vermeiden und somit die Gesamtgröße der Detektorschaltung möglichst klein zu halten. Dadurch können nämlich mehrere Sensorelemente und daran angeordnete Detektorschaltungen möglichst nahe nebeneinander positioniert werden, da kein Bauraum für über die Kanten geführten Leitstrukturen vorgehalten werden braucht.

In einer erfindungsgemäßen Ausführung sind einem Sensorelement mehrere Detektorschaltungen (bspw. vier) zugeordnet. Diese Detektorschaltungen sind dabei rasterartig an der Gegenkontaktfläche des Sensorelements angeordnet und kontaktiert. Jeder ASIC ist dabei mit jeweils einer separat zugeordneten Gruppe von Sensorpixeln kontaktiert.

In einer weiteren erfindungsgemäßen Ausführung umfasst der Photonendetektor mehrere nebeneinander angeordnete Sensorelemente, die eine Sensorfläche des Photonendetektors aufspannen. Entsprechend umfasst der Photonendetektor auch mehrere der vorstehend beschriebenen Detektorschaltungen. Vorzugsweise sind auch in diesem Fall jedem Sensorelement mehrere Detektorschaltungen zugeordnet. Die Gehäuse von wenigstens zwei (insbesondere zueinander benachbarten) Detektorschaltungen, vorzugsweise von wenigstens vier, bevorzugt von einer Vielzahl, optional von allen Detektorschaltungen sind dabei einteilig, insbesondere monolithisch (d. h. aus dem gleichen Material und in einem gemeinsamen Fertigungsprozess ohne Ausbildung von Trennstellen zwischen den einzelnen "gedachten" Gehäusegrenzen) miteinander ausgebildet. D. h. die jeweiligen ASICs sind gemeinsam in ein "Gesamtgehäuse" eingebettet und bilden ein vergleichsweise großflächiges, integrales "Detektorschaltungsarray", sodass vereinzelte Detektorschaltungen in Form separater, gekapselter "Chips" entfallen können. Grundsätzlich ist es dabei möglich, dass mehrere Sensorelemente und die entsprechend zugeordneten Detektorschaltungen in Gruppen (bspw. sogenannte Sensorboards) zusammengefasst werden, deren Detektorschaltungen einteilig miteinander verbunden sind. In diesem Fall sind vorzugsweise mehrere separate Sensorelemente einem solchen Detektorschaltungsarray aufgelegt. Alternativ können aber auch die entsprechenden Sensorelemente einstückig, insbesondere monolithisch miteinander verbunden sein. Durch den Einsatz von solchen Sensorboards (insbesondere unter Nutzung eines integralen Detektorschaltungsarrays) lassen sich beispielsweise vergleichsweise große Detektorflächen durch Aneinanderreihen solcher Sensorboards vergleichsweise einfach herstellen.

Alternativ und/oder in Abhängigkeit von der Größe der gesamten Detektorfläche des Photonendetektors sind alle Detektorschaltungen des Photonendetektors mit einem gemeinsamen Gesamtgehäuse ausgebildet. Insbesondere sind einem solchen "vollflächigen" integralen "Detektorschaltungsarray" wiederum mehrere separate Sensorelemente aufgelegt.

Durch die Gruppierung der Detektorschaltungen mit einem alle oder zumindest Gruppen von Detektorschaltungen umfassenden Gesamtgehäuse kann vorteilhafterweise auch die Präzision bezüglich der Ausrichtung der Sensorelemente zu den jeweiligen Detektorschaltungen gesteigert oder zumindest eine hinreichend präzise Fertigung vereinfacht werden.

Für die Herstellung der vorstehend beschriebenen Gruppen von Detektorschaltungen mit einem Gesamtgehäuse können beispielsweise alle oder zumindest als "gut" geprüfte ASICs, die von einem gemeinsamen Wafer gefertigt sind, in das gemeinsame Gehäuse eingebettet werden. Insbesondere im Fall des Fan-Out Wafer-Level Chip-Scale Packaging kann so optional eine Trennung der einzelnen "Chips" nach einem gemeinsamen Einbetten der ASICs in die Gehäusemasse entfallen. Optional werden dabei gemeinsame Gehäuse in der Größe eines einzelnen (insbesondere aus mehreren Sensorelementen gebildeten) Sensorboards in der Größe von etwa 2 x 4 cm² (das in diesem Fall insbesondere zwei Sensorelemente mit etwa 2 x 2 cm² Fläche und vorzugsweise jeweils mehrere zugeordnete ASICS aufweist) und größer, bis zu Paneelen von der Größe eines vollständigen Röntgendetektors von etwa 40 x 40 cm² ausgebildet, in denen eine entsprechend hohe Zahl von ASICs eingegossen ist.

In einer weiteren bevorzugten Ausführung ist die oder die jeweilige Detektorschaltung zur Erkennung eines sogenannten Charge-Sharing-Ereignisses eingerichtet. Dazu sind jeweils benachbarte Detektorpixel des jeweiligen ASICs untereinander signalübertagungstechnisch verbunden. Dadurch können Zustände, bei denen Ladungsträger, die ein Photon in einem spezifischen Sensorpixel erzeugt, auch von den Detektorpixeln benachbarter Sensorpixel registriert werden, erkannt werden. Vorzugsweise sind die jeweiligen benachbarten Detektorpixel dabei bspw. über Verdrahtungsebenen des typischerweise mittels eines CMOS-Herstellungsprozesses gebildeten, jeweiligen ASICs untereinander kontaktiert. Bei Kenntnis derartiger Charge-Sharing-Ereignisse können diese bei einer Auswertung der einfallenden Strahlung berücksichtigt und insbesondere bei der Erzeugung eines Bildes kompensiert werden. Dies steigert wiederum die Präzision, insbesondere die Orts- und/oder Energieauflösung des erzeugten Bildes. Außerdem können so auch sogenannte K-escapes detektiert werden. Bei diesen wird üblicherweise innerhalb des Sensorelements selbst erneut Strahlung insbesondere durch Wiederauffüllungsvorgänge der sogenannten K-Schale des Halbleitermaterials gebildet, die im selben oder insbesondere benachbarten Sensorpixeln wiederum zur Erzeugung von Ladungsträgern führen kann. Dies kann zu einer Verfälschung der Bilderzeugung führen.

In einer bevorzugten Weiterbildung sind zur Erkennung eines solchen Charge-Sharing-Ereignisses (insbesondere zwischen zwei benachbarten Sensorpixeln) auch randseitig einander zugewandte Detektorpixel zweier ASICs, die in einteilig verbundenen Gehäusen nebeneinander angeordnet sind, untereinander kontaktiert. Die Kontaktierung erfolgt hierbei über die oder eine weitere Umkontaktierungslage der - einteilig miteinander verbundenen - Gehäuse, d. h. des gemeinsamen Gehäuses. Diese Nutzung der Umkontaktierungslage der einteilig miteinander verbundenen Gehäuse (d. h. des gemeinsamen Gehäuses) zur Kontaktierung der einzelnen Detektorpixel untereinander sowie zur Kontaktierung der Detektorpixel über ASIC-Grenzen hinweg ermöglicht dabei vorteilhafterweise eine höhere Verbindungsgeschwindigkeit insbesondere aufgrund vergleichsweise geringer parasitärer Impedanzen insbesondere im Vergleich zur Nutzung von Bond-Drähten und/oder von Through Silicon Vias. Ebenso lassen sich mittels der Umkontaktierungslage die entsprechenden Signalleitungen auch vergleichsweise klein und somit mit hoher Leiterbahndichte ausführen, sodass der gesamte Bauraumbedarf der entsprechenden Detektorschaltung verringert werden kann. Diese Ausführung stellt auch eine eigenständige Erfindung dar

Erfindungsgemäß sind zwei ASICs, die in einteilig verbundenen Gehäusen nebeneinander angeordnet sind, unter Nutzung der oder einer weiteren Umkontaktierungslage ihrer Gehäuse (d. h. ihres Gesamtgehäuses) miteinander signalübertagungstechnisch verbunden. Diese Kontaktierung zweier ASICs untereinander dient - optional zusätzlich zu der vorstehend beschriebenen Erkennung von Charge-Sharing-Ereignissen - beispielsweise zur Taktverteilung zwischen den und/oder zur gemeinsamen Konfiguration der einzelnen ASICs. Dabei wird vorteilhafterweise ebenfalls die geringe parasitäre Impedanz der Leiterbahnen der Umkontaktierungslage, deren geringer Platzbedarf etc. genutzt. Außerdem lässt sich durch die Nutzung der Umkontaktierungslage Leitungsaufwand beispielsweise auf einer räumlich den Detektorschaltungen nachgeordneten Trägerleiterplatte einsparen.

In einer bevorzugten Ausführung findet der vorstehend beschriebene Photonendetektor Einsatz als ein sogenannter Flat-Panel-Detektor bei einem Röntgengerät.

Die Erfindung betrifft auch ein Verfahren zur Herstellung des vorstehend beschriebenen Photonendetektors, insbesondere des Röntgenstrahlungsdetektors. Verfahrensgemäß wird dabei zunächst das vorstehend beschriebene, durch das Halbleitermaterial gebildete und für einfallende Strahlung sensitive zumindest eine Sensorelement bereitgestellt. Dieses zumindest eine Sensorelement bildet dabei die vorstehend beschriebene Pixelmatrix aus der Anzahl, insbesondere der Vielzahl von Sensorpixeln. Des Weiteren wir die vorstehend beschriebene Detektorschaltung bereitgestellt. Die Detektorschaltung wird dem Sensorelement in der Strahlungseinfallsrichtung nachgelagert. D. h. die Detektorschaltung wird insbesondere derart zu dem Sensorelement positioniert, dass sie auf einer einer (Röntgen-) Strahlenquelle abgewandten Seite (als Rückseite bezeichnet) des Sensorelements angeordnet ist. Die Detektorschaltung wird dabei derart gewählt, dass der integrierte Schaltkreis, konkret der vorstehend beschriebene ASIC der Detektorschaltung - der eine zumindest zu einer Teilmenge der Sensorpixel korrespondierende Anzahl an Detektorpixeln aufweist - in seiner Flächenausdehnung die Flächenausdehnung des Sensorelements um mehr als eine Pixelbreite, vorzugsweise um mehr als zwei, drei, vier oder mehr Pixelbreiten der Sensorpixel unterschreitet. Außerdem werden die Detektorpixel des ASICs signalübertragungstechnisch mit den Sensorpixeln (zumindest der zugeordneten Teilmenge an Sensorpixeln) mittels der auf dem Gehäuse, in das der ASIC eingebettet ist, angeordneten Umkontaktierungslage kontaktiert, wobei die Umkontaktierungslage eine dem Sensorelement zugewandte, einem jeden Detektorpixel zugeordnete Kontaktstellen tragende, Kontaktfläche des integrierten Schaltkreises flächig aufspreizt auf eine dem integrierten Schaltkreis zugewandten Gegenkontaktfläche des Sensorelements, und wobei die Umkontaktierungslage durch ein Dünnschicht-Applikationsverfahren ausgebildet ist.

Dabei wird außerdem in einem weiteren Verfahrensschritt, der parallel zum Bereitstellen des wenigstens einen Sensorelements oder zeitlich vor- oder nachgelagert erfolgt, mehrere für ein einzelnes Sensorelement vorgesehene integrierte Schaltkreise in einem gemeinsamen, einteilig ausgebildeten Gehäuse zusammengefasst und in dem Verfahrensschritt des Signalübertragungstechnischen Kontaktierens die durch das gemeinsame Gehäuse zusammengefassten integrierte Schaltkreise an das Sensorelement angebracht und die einander entsprechenden Sensorpixel mit den Detektorpixeln der jeweiligen integrierten Schaltkreises kontaktiert, oder es werden mehr integrierte Schaltkreise als für ein einzelnes Sensorelement erforderlich in einem gemeinsamen, einteilig ausgebildeten Gehäuse zu einem Detektorschaltungsarray zusammengefasst und im Verfahrensschritt des Signalübertragungstechnischen Kontaktierens werden mehrere einzelne Sensorelemente auf dieses Detektorschaltungsarray aufgelegt. Dabei sind zwei integrierte Schaltkreise, die in dem gemeinsamen Gehäuse nebeneinander angeordnet sind, sind unter Nutzung der oder einer weiteren Umkontaktierungslage des gemeinsamen Gehäuses miteinander signalübertragungstechnisch verbunden.

Dem Verfahren zur Herstellung des Photonendetektors kommen insbesondere die sich aus den Merkmalen des vorstehend beschriebenen Photonendetektors ergebenden Vorteile ebenfalls zu.

In einer bevorzugten Ausführung wird eine jeweilige Detektorschaltung mit dem ASIC, vorzugsweise ausgehend von dem ASIC mittels des als Fan-Out Wafer-Level Chip-Scale Packaging bezeichneten Verfahrens gefertigt. In diesem Fall sind die Detektorpixel somit vorteilhafterweise signalübertagungstechnisch mittels der vorstehend beschriebenen Umkontaktierungsschicht - und vorzugsweise mittels auf diese applizierten "Lötbällen" oder "Solderballs" - elektrisch mit den Sensorpixeln kontaktiert.

Die Erfindung betrifft auch ein medizinisches Röntgengerät, das den vorstehend beschriebenen Photonendetektor, vorzugsweise den durch diesen gebildeten Röntgenstrahlungsdetektor aufweist. Beispielsweise handelt es sich bei diesem Röntgengerät um einen Computertomographen, ein C-Bogen-Röntgengerät oder dergleichen.

Die Erfindung betrifft außerdem ein medizinisches Röntgengerät, das den vorstehend beschriebenen Photonendetektor, vorzugsweise den durch diesen gebildeten Röntgenstrahlungsdetektor aufweist, wobei das Röntgengerät ein Computertomograph ist.

Die Konjunktion "und/oder" ist hier und im Folgenden insbesondere derart zu verstehen, dass die mittels dieser Konjunktion verknüpften Merkmale sowohl gemeinsam als auch als Alternativen zueinander ausgebildet sein können.

Nachfolgend werden Ausführungsbeispiele der Erfindung anhand einer Zeichnung näher dargestellt. Darin zeigen:
- FIG 1: in einer schematischen Seitenansicht ein Röntgengerät mit einer Röntgenstrahlenquelle und einem Röntgendetektor,
- FIG 2: in einem schematischen Querschnitt einen Ausschnitt des Röntgenstrahlungsdetektors,
- FIG 3: in einer schematischen Ansicht auf eine Oberseite ein Sensorelement des Röntgenstrahlungsdetektors,
- FIG 4: in Ansicht gemäß FIG 2 ein weiteres Ausführungsbeispiel des Röntgenstrahlungsdetektors,
- FIG 5: in Ansicht gemäß FIG 3 wiederum ein weiteres Ausführungsbeispiel des Röntgenstrahlungsdetektors,
- FIG 6: in einer schematischen Ansicht ein weiteres Ausführungsbeispiel des Röntgengeräts in Form eines Computertomographen, und
- FIG 7: in einem schematischen Ablaufdiagram ein Verfahren zur Herstellung des Röntgenstrahlungsdetektors.

Einander entsprechende Teile sind in allen Figuren stets mit gleichen Bezugszeichen versehen.

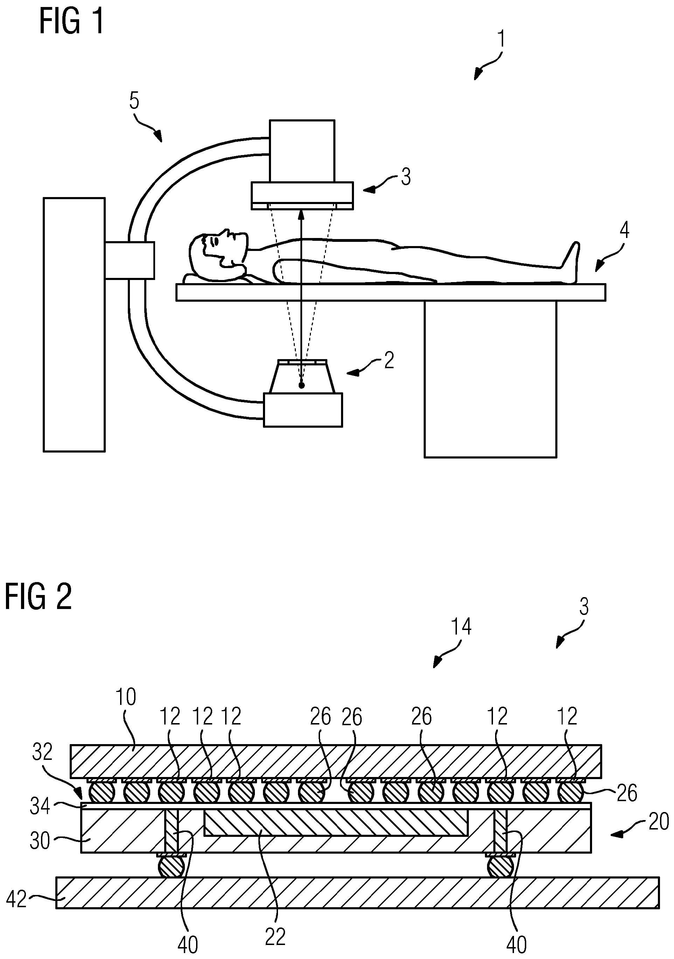

In FIG 1 ist ein Röntgengerät 1 schematisch dargestellt. Konkret handelt es sich bei diesem Röntgengerät 1 um ein sogenanntes C-Bogen-Röntgengerät. Das Röntgengerät 1 umfasst eine Röntgenstrahlenquelle 2, die in Gegenüberstellung zu einem Röntgenstrahlungsdetektor (kurz: Röntgendetektor 3) angeordnet ist. Der Röntgendetektor 3 bildet einen Photonendetektor zur Erfassung von Röntgen-Photonen, die von der Röntgenstrahlenquelle 2 ausgehen. Der Röntgendetektor 3 ist dabei als sogenannte Flat-Panel Detektor ausgebildet. Das Röntgengerät 1 umfasst außerdem eine Patientenliege 4 sowie eine Handhabungseinheit 5, an der das aus der Röntgenstrahlenquelle 2 und dem Röntgendetektor 3 gebildete Röntgensystem um die Patientenliege 4 rotierbar angeordnet ist.

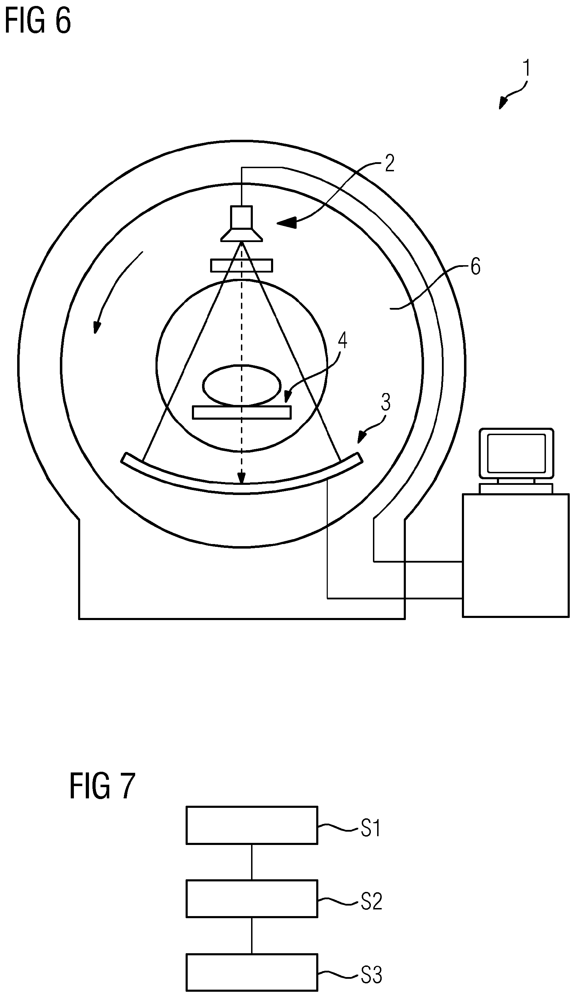

In FIG 6 ist ein Röntgengeräts 1, welches einen Computertomographen umfasst, dargestellt. Das Röntgengerät 1 bildet dabei einen Computertomographen, wobei das Röntgensystem an einem Drehkranz 6 um die Patientenliege 4 rotierbar gehaltert ist. Die Patientenliege 4 ist entlang ihrer Längserstreckung in einen durch den Drehkranz 6 gebildeten Tunnel einschiebbar.

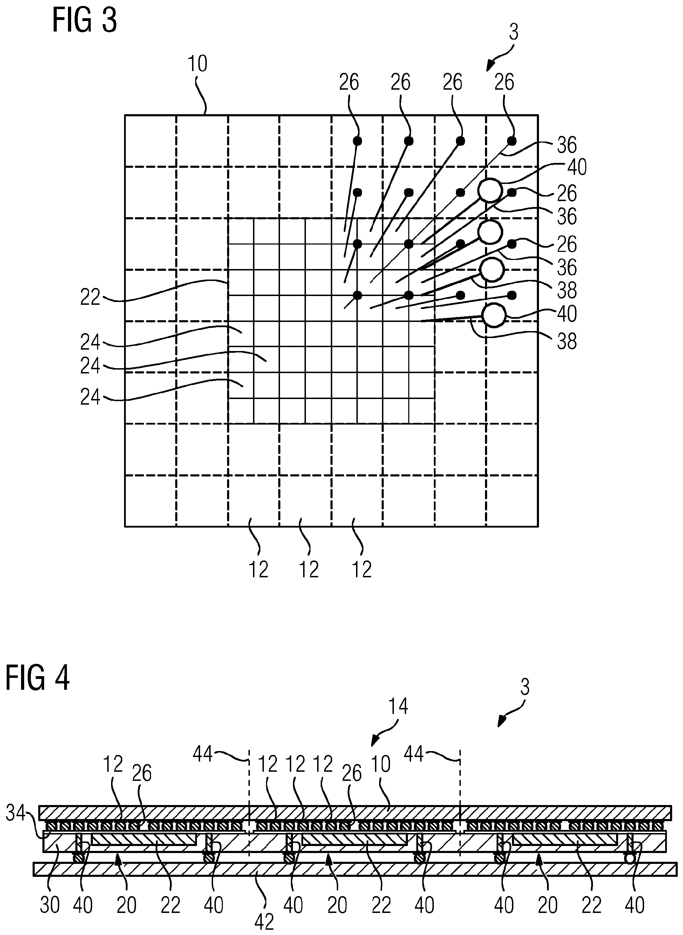

Der Röntgendetektor 3 ist (sowohl bei dem C-Bogen-Röntgengerät als auch bei dem Computertomographen) als sogenannter photonen- oder quantenzählender Detektor ausgebildet. Dazu umfasst der Röntgendetektor 3 ein aus einem für einfallende Röntgenstrahlung sensitiven Material, konkret einem Halbleitermaterial, hier Cadmium-Tellurid, gebildetes Sensorelement 10. Konkret ist dieses Sensorelement 10 durch eine Platte aus diesem Halbleitermaterial gebildet. Außerdem bildet das Sensorelement 10 eine Pixelmatrix aus einer Vielzahl von einzelnen Sensorpixeln 12 aus. Wie in FIG 3 schematisch dargestellt, sind die einzelnen Sensorpixel 12 dabei rasterartig (also regelmäßig) angeordnet. Konkret werden die Sensorpixel 12 durch voneinander separate Kontaktstellen, die auf eine einer Strahlungseinfallseite 14 des Sensorelements 10 abgewandten Rückseite appliziert sind, gebildet (siehe FIG 2).

Durch in das Sensorelement 10 einfallende Röntgen-Photonen werden in dem Halbleitermaterial des Sensorelements 10 korrespondierende Ladungsträger, nämlich Elektronen-Loch-Paare gebildet. Aufgrund einer zwischen der Strahlungseinfallseite 14 und der Rückseite angelegten Hochspannung driften die Ladungsträger (zumindest die negativen oder positiven Ladungsträger - je nach angelegter Hochspannung) zur Rückseite und können dort mittels der den jeweiligen Sensorpixeln 12 zugeordneten Kontaktstellen detektiert werden.

Zur Registrierung, d. h. Erfassung der jeweiligen Ladungsträger umfasst der Röntgendetektor 3 eine dem Sensorelement 10 nachgelagerte Detektorschaltung 20. Diese Detektorschaltung 20 dient neben der Erfassung der Ladungsträger auch zur Vorverarbeitung der bei der Erfassung der einzelnen Ladungsträger generierten Sensorsignale (bspw. durch eine Art Filterung mittels eines Schwellwertvergleichs). Dazu umfasst die Detektorschaltung 20 einen integrierten Schaltkreis, konkret einen ASIC 22. Der ASIC 22 weist dabei in einer Variante für jedes Sensorpixel 12 des zugeordneten Sensorelements 10 einen korrespondierenden Detektorpixel 24 auf (siehe FIG 3). Jeder der Detektorpixel 24 ist dabei signalübertagungstechnisch mit jeweils einem zugeordneten Sensorpixel 12 kontaktiert. In FIG 2 ist diese Kontaktierung beispielhaft durch einzelne Solderballs (auch: "Lötbälle 26") angedeutet.

Um den jeweiligen ASIC 22 möglichst kompakt und dadurch kostengünstig fertigen zu können, unterschreitet eine Flächenausdehnung des ASICs 22, konkret eine Kantenlänge des ASICs 22 die Flächenausdehnung des Sensorelements 10, konkret deren entsprechende Kantenlänge um mindestens eine Pixelbreite eines Sensorpixels 12. Wie in FIG 3 dargestellt ist, unterschreitet die Kantenlänge des ASICs 22 die Kantenlänge des Sensorelements 10 um vier Pixelbreiten der Sensorpixel 12. Um die einzelnen Detektorpixel 24 dennoch mit den über die Fläche des ASICs 22 überstehenden Sensorpixeln 12 kontaktieren zu können, weist die Detektorschaltung 20 außerdem ein Gehäuse 30 auf, in das der ASIC 22 eingebettet ist. Konkret handelt es sich bei dem Gehäuse 30 um einen "Block" aus Einbettmasse, konkret ein Gießharz, in das der ASIC 22 oberflächenbündig eingebettet ist. Zur Kontaktierung, konkret zur Leitungsumverteilung von den einzelnen Detektorpixeln 24 zu den einzelnen Sensorpixeln 12 ist auf eine dem Sensorelement 10 zugewandte Pixelseite 32 der Detektorschaltung 20 eine Umkontaktierungslage 34 appliziert. Die Umkontaktierungslage 34 weist wie in FIG 3 dargestellt, eine Anzahl von Leiterbahnen 36 auf, die von den einzelnen Detektorpixeln 24 zu den Lötbällen 26 führen. Außerdem weist die Umkontaktierungslage 34 auch Leiterbahnen 38 auf, die von weiteren Schaltungsstrukturen (nicht näher dargestellt) des ASICs 22 zu Durchkontaktierungen 40 führen. Die Durchkontaktierungen 40 sind dabei im Gehäuse 30 selbst ausgebildet und führen somit lediglich durch Kunststoff-Masse hindurch. Die Durchkontaktierungen 40 dienen dabei zum Anschluss (d. h. zur signalübertragungstechnischen Kontaktierung) des ASICs 22 mit nachgeordneten Signalführungsmitteln, die im vorliegenden Ausführungsbeispiel durch eine Leiterplatte 42 gebildet sind.

Die Umkontaktierungslage 34 ist unter anderem durch Dampfphasenabscheidungsprozesse auf die Pixelseite 32 der Detektorschaltung 20 aufgebracht. Die Detektorschaltung 20 ist durch das sogenannte Fan-Out Wafer-Level Chip-Scale Packaging-Verfahren hergestellt.

In FIG 4 ist ein weiteres Ausführungsbeispiel näher dargestellt. In diesem Fall weist das Sensorelement 10 des Röntgendetektors 3 eine gegenüber einem einzelnen ASIC 22 erhöhte Anzahl an Sensorpixeln 12 und deshalb mehrere zugeordnete Detektorschaltungen 20 auf. Die jeweils zugeordneten Detektorschaltungen 20 sind in diesem Fall mittels ihrer Gehäuse 30 einstückig miteinander verbunden. D. h. die in FIG 4 im Querschnitt dargestellten ASICs 22 sind in einem gemeinsamen Gehäuse 30 eingebettet. Die "gedachten" Trennlinien 44 zwischen den einzelnen Detektorschaltungen 20 sind in FIG 4 angedeutet. Durch die Nutzung des gemeinsamen Gehäuses 30 für mehrere ASICs 22 kann die Präzision der Ausrichtung der einzelnen ASICs 22 zueinander (und bei mehreren benachbarten Sensorelementen 10 somit auch dieser Sensorelemente 10 zueinander) erhöht werden.

In einem optionalen, nicht näher dargestellten Ausführungsbeispiel (ebenfalls beschrieben anhand von FIG 4) sind mehrere separate Sensorelemente 10 auf die in dem gemeinsamen Gehäuse 30 eingebetteten ASICs 22 appliziert.

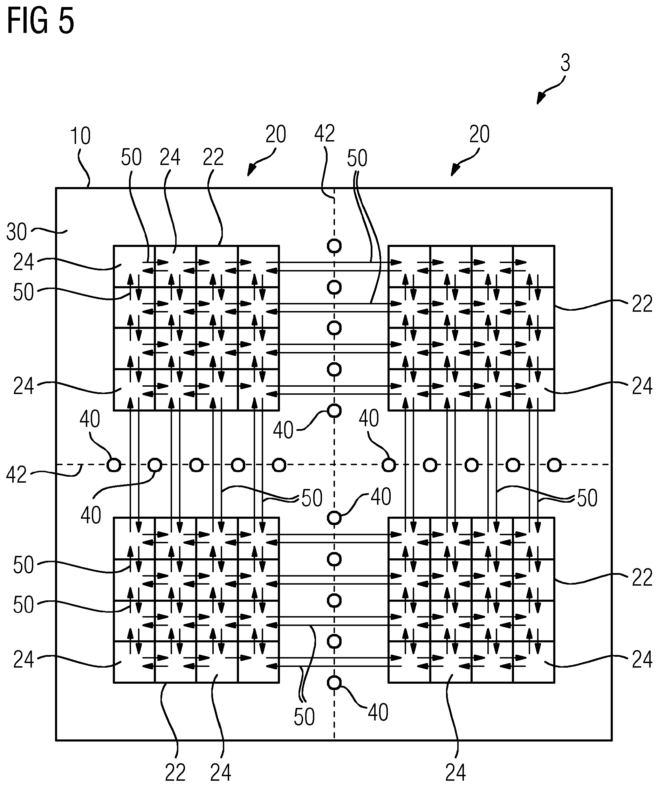

In FIG 5 ist eine Weiterbildung des in FIG 4 beschriebenen Röntgendetektors 3 dargestellt. Der Röntgendetektor 3 ist dabei zur Erkennung von sogenannten Charge-Sharing-Ereignissen eingerichtet und vorgesehen. Dazu sind die einzelnen Detektorpixel 24 eines jeden ASICs 22, konkret die nebeneinander angeordneten Detektorpixel 24 untereinander signalübertagungstechnisch kontaktiert. In FIG 5 ist diese Kontaktierung durch jeweils ein Paar gegengleich gerichteter Pfeile 50 angedeutet. In einem optionalen, nicht näher dargestellten Ausführungsbeispiel sind auch die diagonal benachbarten Sensorpixel 12 untereinander kontaktiert. Im in FIG 5 dargestellten Ausführungsbeispiel ist die Umkontaktierungslage 34 über die gedachten Trennlinien 44 zwischen den jeweiligen Detektorschaltungen 20 hinweg über das gesamte gemeinsame Gehäuse 30 aller in diesem eingebetteten ASICs 22 (im dargestellten Ausführungsbeispiel 2 x 2 ASICs 22) hinweg erstreckt. Um nun auch Charge-Sharing-Ereignisse zwischen den Sensorpixeln 12 zueinander benachbarter separat voneinander gebildeter Sensorelemente 10 erkennen zu können, sind die Detektorpixel 24 einander benachbarter ASICs 22 mittels der Umkontaktierungslage 34 ebenfalls miteinander signalübertragungstechnisch verschaltet. Dadurch kann die Erkennungsgenauigkeit für Charge-Sharing-Ereignisse erhöht und somit die Präzision des Röntgendetektors 3 im bestimmungsgemäßen Betrieb des Röntgengeräts 1 erhöht werden.

In FIG 7 ist schematisch ein Verfahren zur Herstellung des Röntgendetektors 3, aufweisend wenigstens ein Sensorelement 10 und eine dem Sensorelement 10 in Strahlungseinfallsrichtung nachgelagerte Detektorschaltung 20, dargestellt.

Zunächst wird in einem Schritt S1 wenigstens ein Sensorelement 10 bereitgestellt, wobei das Sensorelement 10 durch ein Halbleitermaterial gebildet und für einfallende Strahlung sensitiv ist, und wobei das Sensorelement 10 eine Pixelmatrix mit einer Anzahl von Sensorpixeln 12 bildet. In einem weiteren Verfahrensschritt S2 wird die Detektorschaltung 20 bereitgestellt, wobei die Detektorschaltung 20 zur Erfassung von in dem Halbleitermaterial des Sensorelements 10 strahlungsbedingt generierten Ladungsträgern dient und einen integrierten Schaltkreis 22 mit einer Anzahl an Detektorpixeln 24 aufweist, wobei eine Flächenausdehnung des integrierten Schaltkreises 22 die Flächenausdehnung des Sensorelements 10 um mehr als eine Pixelbreite der Sensorpixel 12 unterschreitet.

In einem weiteren Schritt S3 werden die Detektorpixel 24 signalübertragungstechnisch mit den Sensorpixeln 12 mittels einer auf einem Gehäuse 30, in das der integrierte Schaltkreis 22 eingebettet ist, angeordneten Umkontaktierungslage 34 kontaktiert.

In einem weiteren Verfahrensschritt- der parallel zum Verfahrensschritt S1 oder zeitlich vor- oder nachgelagert erfolgen kann - können in einer ersten erfindungsgemäßen Variante mehrere für ein einzelnes Sensorelement 10 vorgesehene ASICs 22 (wie vorstehend bspw. anhand von FIG 4 beschrieben) in dem gemeinsamen Gehäuse 30 zusammengefasst werden. In dem Verfahrensschritt S3 werden dann die durch das gemeinsame Gehäuse 30 zusammengefassten ASICs 22 an das Sensorelement 10 angebracht und die, einander entsprechenden Sensorpixel 12 mit den Detektorpixeln der jeweiligen ASICs 22 (mittelbar mittels der Lötbälle 26 und der Leiterbahnen 36 der Umkontaktierungslage 34) kontaktiert.

In einer zweiten erfindungsgemäßen Variante des vorstehend beschriebenen Herstellungsverfahrens werden mehr ASICs 22 als für ein einzelnes Sensorelement 10 erforderlich (bspw. die 4-fache, 6-fache, 10-fache oder größere Anzahl) in dem gemeinsamen Gehäuse 30 zu einem "Detektorschaltungsarray" zusammengefasst. Im Verfahrensschritt S3 werden dann mehrere einzelne Sensorelemente 10 (also beispielsweise 4, 6, 10 etc.) auf dieses Detektorschaltungsarray aufgelegt. Die Kontaktierung der jeweiligen Sensorpixel 12 mit den Detektorpixeln der einzelnen ASICs 22 erfolgt dann wiederum analog zu der vorstehenden Beschreibung.

## Patentansprüche

1. Photonendetektor (3), insbesondere Röntgenstrahlungsdetektor (3),
- mit einem durch ein Halbleitermaterial gebildeten, für einfallende Strahlung sensitiven Sensorelement (10), das eine Pixelmatrix mit einer Anzahl von Sensorpixeln (12) bildet,
- mit einer Detektorschaltung (20), die dem Sensorelement (10) in Strahlungseinfallsrichtung nachgelagert ist und die zur Erfassung von in dem Halbleitermaterial des Sensorelements (10) strahlungsbedingt generierten Ladungsträgern dient, wobei die Detektorschaltung (20) einen integrierten Schaltkreis (22) mit einer Anzahl an Detektorpixeln (24), die signalübertragungstechnisch mit den Sensorpixeln (12) kontaktiert sind, umfasst,
wobei eine Flächenausdehnung des integrierten Schaltkreises (22) die Flächenausdehnung des Sensorelements (10) um mehr als eine Pixelbreite der Sensorpixel (12) unterschreitet, wobei die Detektorschaltung (20) ein den integrierten Schaltkreis (22) umgebendes Gehäuse (30) aufweist, in das der integrierte Schaltkreis (22) eingebettet ist und auf dem auf einer dem Sensorelement (10) zugewandten Pixelseite (32) eine Umkontaktierungslage (34) ausgebildet ist, in der Kontaktstellen zur signalübertragungstechnischen Verbindung der Detektorpixel (24) mit den entsprechend zugeordneten Sensorpixeln (12) sowie Leiterbahnen (36,38) zur Verbindung der Kontaktstellen mit den Detektorpixeln (24) des integrierten Schaltkreises (22) ausgebildet sind, wobei die Umkontaktierungslage (34) eine dem Sensorelement (10) zugewandte, einem jeden Detektorpixel (24) zugeordnete Kontaktstellen tragende, Kontaktfläche des integrierten Schaltkreises (22) flächig aufspreizt auf eine dem integrierten Schaltkreis (22) zugewandten Gegenkontaktfläche des Sensorelements (10), und wobei die Umkontaktierungslage (34) durch ein Dünnschicht-Applikationsverfahren ausgebildet ist, und
wobei dem Sensorelement (10) mehrere Detektorschaltungen (20) umfassend jeweils einen integrierten Schaltkreis (22) zugeordnet sind, welche rasterartig an der Gegenkontaktfläche des Sensorelements (10) angeordnet und kontaktiert sind, so dass jeder integrierte Schaltkreis (22) mit jeweils einer separat zugeordneten Gruppe von Sensorpixeln (12) kontaktiert ist und wobei die Gehäuse (30) von wenigstens zwei Detektorschaltungen (20) umfassend jeweils einen integrierten Schaltkreis (22) einteilig, insbesondere monolithisch, miteinander ausgebildet sind,
**dadurch gekennzeichnet, dass**
zwei integrierte Schaltkreise (22), die mit einteilig verbundenen Gehäusen (30) nebeneinander angeordnet sind, unter Nutzung der oder einer weiteren Umkontaktierungslage (34) der Gehäuse (30) miteinander signalübertragungstechnisch verbunden sind.

2. Photonendetektor (3), insbesondere Röntgenstrahlungsdetektor (3),
- mit mehreren durch ein Halbleitermaterial gebildeten, für einfallende Strahlung sensitiven Sensorelementen (10), welche jeweils eine Pixelmatrix mit einer Anzahl von Sensorpixeln (12) bilden,
wobei die mehreren Sensorelemente (10) nebeneinander angeordnet sind und eine Sensorfläche des Photonendetektors (3) aufspannen,
- mit mehreren, den Sensorelementen (10) entsprechend zugeordneten, Detektorschaltungen, die den Sensorelementen (10) in Strahlungseinfallsrichtung jeweils nachgelagert sind und die zur Erfassung von in dem Halbleitermaterial der Sensorelemente (10) strahlungsbedingt generierten Ladungsträgern dienen, wobei die Detektorschaltungen (20) jeweils einen integrierten Schaltkreis (22) mit einer Anzahl an Detektorpixeln (24), die signalübertragungstechnisch mit den Sensorpixeln (12) kontaktiert sind, umfassen,
wobei eine Flächenausdehnung des jeweiligen integrierten Schaltkreises (22) die Flächenausdehnung des jeweiligen Sensorelements (10) um mehr als eine Pixelbreite der Sensorpixel (12) unterschreitet,
wobei die Detektorschaltungen (20) jeweils ein den integrierten Schaltkreis (22) umgebendes Gehäuse (30) aufweisen, in das der integrierte Schaltkreis (22) eingebettet ist und auf dem auf einer dem zugeordneten Sensorelement (10) zugewandten Pixelseite (32) eine Umkontaktierungslage (34) ausgebildet ist, in der Kontaktstellen zur signalübertragungstechnischen Verbindung der Detektorpixel (24) mit den entsprechend zugeordneten Sensorpixeln (12) sowie Leiterbahnen (36,38) zur Verbindung der Kontaktstellen mit den Detektorpixeln (24) des integrierten Schaltkreises (22) ausgebildet sind,
wobei die Umkontaktierungslage (34) eine dem jeweiligen Sensorelement (10) zugewandte, einem jeden Detektorpixel (24) zugeordnete Kontaktstellen tragende, Kontaktfläche des integrierten Schaltkreises (22) flächig aufspreizt auf eine dem integrierten Schaltkreis (22) zugewandten Gegenkontaktfläche des jeweiligen Sensorelements (10), und wobei die Umkontaktierungslage (34) durch ein Dünnschicht-Applikationsverfahren ausgebildet ist,
und wobei die Gehäuse (30) von wenigstens zwei Detektorschaltungen (20) umfassend jeweils einen integrierten Schaltkreis (22) einteilig, insbesondere monolithisch, miteinander ausgebildet sind, **dadurch gekennzeichnet, dass** zwei integrierte Schaltkreise (22), die mit einteilig verbundenen Gehäusen (30) nebeneinander angeordnet sind, unter Nutzung der oder einer weiteren Umkontaktierungslage (34) der Gehäuse (30) miteinander signalübertragungstechnisch verbunden sind.

3. Photonendetektor (3) nach Anspruch 1 oder 2,
wobei ein jeweiliger integrierter Schaltkreis (22) oberflächenbündig und an seinen Kanten spaltfrei in das Gehäuse (30) eingebettet ist.

4. Photonendetektor (3) nach einem der Ansprüche 1 bis 3,
wobei eine jeweilige Detektorschaltung (20) durch den integrierten Schaltkreis (22) mittels Fan-Out Wafer-Level Chip-Scale Packaging gefertigt ist.

5. Photonendetektor (3) nach einem der Ansprüche 1 bis 4,
wobei eine jeweilige Detektorschaltung (20) im Bereich des Gehäuses (30) wenigstens eine Durchkontaktierung (40) zur signalübertragungstechnischen Anbindung an eine nachgelagerte Steuer- und Auswerteeinheit und/oder an nachgelagerte Signalführungsmittel (42) aufweist.

6. Photonendetektor (3) nach einem der Ansprüche 1 bis 5,
wobei eine jeweilige Detektorschaltung (20) zur Erkennung eines Charge-Sharing-Ereignisses eingerichtet ist, indem jeweils benachbarte Detektorpixel (24) des jeweiligen integrierten Schaltkreises (22) untereinander signalübertragungstechnisch verbunden sind.

7. Photonendetektor (3) nach einem der Ansprüche 1 bis 6,
wobei zur Erkennung eines Charge-Sharing-Ereignisses randseitig einander zugewandte Detektorpixel (24) zweier integrierter Schaltkreise (22), die in einteilig verbundenen Gehäusen (30) nebeneinander angeordnet sind, über die oder eine weitere Umkontaktierungslage (34) der Gehäuse (30) miteinander signalübertragungstechnisch verbunden sind.

8. Verfahren zur Herstellung eines Photonendetektors (3), insbesondere eines Röntgenstrahlungsdetektors (3), aufweisend ein Sensorelement (10) und eine dem Sensorelement (10) in Strahlungseinfallsrichtung nachgelagerte Detektorschaltung (20), umfassend die Schritte
- Bereitstellen (S1) des Sensorelements (10), wobei das Sensorelement (10) durch ein Halbleitermaterial gebildet und für einfallende Strahlung sensitiv ist, und wobei das Sensorelement (10) eine Pixelmatrix mit einer Anzahl von Sensorpixeln (12) bildet,
- Bereitstellen (S2) der Detektorschaltung (20), wobei die Detektorschaltung (20) zur Erfassung von in dem Halbleitermaterial des Sensorelements (10) strahlungsbedingt generierten Ladungsträgern dient und einen integrierten Schaltkreis (22) mit einer Anzahl an Detektorpixeln (24) aufweist, wobei eine Flächenausdehnung des integrierten Schaltkreises (22) die Flächenausdehnung des Sensorelements (10) um mehr als eine Pixelbreite der Sensorpixel (12) unterschreitet, und
- Signalübertragungstechnisches Kontaktieren (S3) der Detektorpixel (24) mit den Sensorpixeln (12) mittels einer auf einem Gehäuse (30), in das der integrierte Schaltkreis (22) eingebettet ist, angeordneten Umkontaktierungslage (34), wobei die Umkontaktierungslage (34) eine dem Sensorelement (10) zugewandte, einem jeden Detektorpixel (24) zugeordnete Kontaktstellen tragende, Kontaktfläche des integrierten Schaltkreises (22) flächig aufspreizt auf eine dem integrierten Schaltkreis (22) zugewandten Gegenkontaktfläche des Sensorelements (10), und wobei die Umkontaktierungslage (34) durch ein Dünnschicht-Applikationsverfahren ausgebildet ist,
**dadurch gekennzeichnet, dass**
in einem weiteren Verfahrensschritt, der parallel zum Bereitstellen (S1) des wenigstens einen Sensorelements oder zeitlich vor- oder nachgelagert erfolgt, mehrere für das Sensorelement (10) vorgesehene integrierte Schaltkreise (22) in einem gemeinsamen, einteilig ausgebildeten Gehäuse (30) zusammengefasst werden und in dem Verfahrensschritt des signalübertragungstechnischen Kontaktierens (S3) die durch das gemeinsame Gehäuse (30) zusammengefassten integrierte Schaltkreise (22) an das Sensorelement (10) angebracht und die einander entsprechenden Sensorpixel (12) mit den Detektorpixeln der jeweiligen integrierten Schaltkreises (22) kontaktiert werden und wobei zwei integrierte Schaltkreise (22), die in dem gemeinsamen Gehäuse (30) nebeneinander angeordnet sind, unter Nutzung der oder einer weiteren Umkontaktierungslage (34) des gemeinsamen Gehäuses (30) miteinander signalübertragungstechnisch verbunden sind.

9. Verfahren zur Herstellung eines Photonendetektors (3), insbesondere eines Röntgenstrahlungsdetektors (3), aufweisend mehrere Sensorelemente (10) und jeweils einer den Sensorelementen (10) in Strahlungseinfallsrichtung nachgelagerten Detektorschaltung (20), umfassend die Schritte
- Bereitstellen (S1) der mehreren Sensorelemente (10), wobei die Sensorelemente (10) jeweils durch ein Halbleitermaterial gebildet und für einfallende Strahlung sensitiv sind, und wobei die Sensorelemente (10) jeweils eine Pixelmatrix mit einer Anzahl von Sensorpixeln (12) bilden,
wobei die mehreren Sensorelemente (10) nebeneinander angeordnet sind und eine Sensorfläche des Photonendetektors (3) aufspannen,
- Bereitstellen (S2) der Detektorschaltungen (20), wobei die Detektorschaltungen (20) zur Erfassung von in dem Halbleitermaterial des jeweiligen Sensorelements (10) strahlungsbedingt generierten Ladungsträgern dienen und jeweils einen integrierten Schaltkreis (22) mit einer Anzahl an Detektorpixeln (24) aufweisen, wobei eine Flächenausdehnung des jeweiligen integrierten Schaltkreises (22) die Flächenausdehnung des jeweiligen Sensorelements (10) um mehr als eine Pixelbreite der Sensorpixel (12) unterschreitet, und
- signalübertragungstechnisches Kontaktieren (S3) der Detektorpixel (24) mit den Sensorpixeln (12) mittels einer auf einem Gehäuse (30), in das der jeweilige integrierte Schaltkreis (22) eingebettet ist, angeordneten Umkontaktierungslage (34), wobei die Umkontaktierungslage (34) eine dem jeweiligen Sensorelement (10) zugewandte, einem jeden Detektorpixel (24) zugeordnete Kontaktstellen tragende, Kontaktfläche des integrierten Schaltkreises (22) flächig aufspreizt auf eine dem integrierten Schaltkreis (22) zugewandten Gegenkontaktfläche des jeweiligen Sensorelements (10), und wobei die Umkontaktierungslage (34) durch ein Dünnschicht-Applikationsverfahren ausgebildet ist,
wobei mehr integrierte Schaltkreise (22) als für ein einzelnes Sensorelement (10) erforderlich in einem gemeinsamen, einteilig ausgebildeten Gehäuse (30) zu einem Detektorschaltungsarray zusammengefasst werden und im Verfahrensschritt des signalübertragungstechnischen Kontaktierens (S3) mehrere einzelne Sensorelemente (10) auf dieses Detektorschaltungsarray aufgelegt werden,
**dadurch gekennzeichnet, dass**
zwei integrierte Schaltkreise (22), die in dem gemeinsamen Gehäuse (30) nebeneinander angeordnet sind, unter Nutzung der oder einer weiteren Umkontaktierungslage (34) des gemeinsamen Gehäuses (30) miteinander signalübertragungstechnisch verbunden sind.

10. Verfahren nach Anspruch 8 oder 9,
wobei eine jeweilige Detektorschaltung (20) mit dem integrierten Schaltkreis (22) mittels Fan-Out Wafer-Level Chip-Scale Packaging gefertigt wird.

11. Medizinisches Röntgengerät (1) mit einem Photonendetektor, insbesondere Röntgenstrahlungsdetektor (3) nach einem der Ansprüche 1 bis 7.

12. Medizinisches Röntgengerät (1) nach Anspruch 11, wobei das Röntgengerät (1) ein Computertomograph ist.

## Claims

1. Photon detector (3), in particular X-ray radiation detector (3),
- comprising one sensor element (10), which is formed by a semiconductor material and is sensitive to incident radiation, and forms a pixel array comprising a number of sensor pixels (12),
- comprising a detector circuit (20), which is situated after the sensor element (10) in the direction of incident radiation, and which is used to detect charge carriers generated in the semiconductor material of the sensor element (10) as a result of radiation, wherein the detector circuit (20) comprises an integrated circuit (22) having a number of detector pixels (24), which are in signal communication contact with the sensor pixels (12),
wherein a surface dimension of the integrated circuit (22) is less than the surface dimension of the sensor element (10) by more than one pixel width of the sensor pixels (12), wherein the detector circuit (20) comprises an enclosure (30), which surrounds the integrated circuit (22) and in which the integrated circuit (22) is embedded, and on which is formed on a pixel face (32) that faces the sensor element (10) a contact redistribution layer (34), in which contact pads are formed for signal-communicatively connecting the detector pixels (24) to the correspondingly assigned sensor pixels (12), and also conductor tracks (36, 38) for connecting the contact pads to the detector pixels (24) of the integrated circuit (22), wherein the contact redistribution layer (34) expands, in a two-dimensional manner, a contact area of the integrated circuit (22), which faces the sensor element (10) and carries contact pads assigned to each detector pixel (24), on a mating contact area of the sensor element (10) which faces the integrated circuit (22), and wherein the contact redistribution layer (34) is formed by a thin film application process,
and wherein a plurality of detector circuits (20), each comprising an integrated circuit (22), are assigned to the sensor element (10) and are arranged on the mating contact area of the sensor element (10) and make contact therewith in a grid formation, so that each integrated circuit (22) makes contact with a separately assigned group of sensor pixels (12) in each case, and wherein the enclosures (30) of at least two detector circuits (20), each comprising an integrated circuit (22), are formed together integrally, in particular monolithically,
**characterised in that**
two integrated circuits (22) arranged side by side by integrally joined enclosures (30) are signal-communicatively connected to one another using the, or an additional, contact redistribution layer (34) of the enclosure (30).

2. Photon detector (3), in particular X-ray radiation detector (3),
- comprising a plurality of sensor elements (10), which are formed by a semiconductor material and are sensitive to incident radiation, and each form a pixel array comprising a number of sensor pixels (12),
wherein the plurality of sensor elements (10) are arranged side by side and span a sensor area of the photon detector (3),
- comprising a plurality of detector circuits, which are assigned to the sensor elements (100) accordingly, are each situated after the sensor elements (10) in the direction of incident radiation, and which are used to detect charge carriers generated in the semiconductor material of the sensor elements (10) as a result of radiation, wherein the detector circuits (20) each comprise an integrated circuit (22) having a number of detector pixels (24), which are in signal communication contact with the sensor pixels (12),
wherein a surface dimension of the respective integrated circuit (22) is less than the surface dimension of the associated sensor element (10) by more than one pixel width of the sensor pixels (12),
wherein the detector circuits (20) each comprise an enclosure (30), which surrounds the integrated circuit (22) and in which the integrated circuit (22) is embedded, and on which is formed on a pixel face (32) that faces the assigned sensor element (10) a contact redistribution layer (34), in which contact pads are formed for signal-communicatively connecting the detector pixels (24) to the correspondingly assigned sensor pixels (12), and also conductor tracks (36, 38) for connecting the contact pads to the detector pixels (24) of the integrated circuit (22), wherein the contact redistribution layer (34) expands, in a two-dimensional manner, a contact area of the integrated circuit (22), which faces the associated sensor element (10) and carries contact pads assigned to each detector pixel (24), on a mating contact area of the associated sensor element (10) which faces the integrated circuit (22), and wherein the contact redistribution layer (34) is formed by a thin film application process,
and wherein the enclosures (30) of at least two detector circuits (20), each comprising an integrated circuit (22), are formed together integrally, in particular monolithically, **characterised in that**
two integrated circuits (22) arranged side by side by integrally joined enclosures (30) are signal-communicatively connected to one another using the, or an additional, contact redistribution layer (34) of the enclosures (30).

3. Photon detector (3) according to claim 1 or 2,
wherein a respective integrated circuit (22) is embedded in the enclosure (30) flush with the surface and without any gaps along its edges.

4. Photon detector (3) according to one of claims 1 to 3,
wherein a respective detector circuit (20) through the integrated circuit (22) is fabricated by fan-out wafer-level chip-scale packaging.

5. Photon detector (3) according to one of claims 1 to 4,
wherein a respective detector circuit (20) comprises in the region of the enclosure (30) at least one via (40) for the signal communication connection to a subsequent control and analysis unit and/or subsequent signal-routing means (42).

6. Photon detector (3) according to one of claims 1 to 5,
wherein a respective detector circuit (20) is configured to identify a charge-sharing event, by the fact that adjacent detector pixels (24) of the associated integrated circuit (22) are signal-communicatively interconnected.

7. Photon detector (3) according to one of claims 1 to 6,
wherein in order to identify a charge-sharing event, detector pixels (24) of two integrated circuits (22) arranged side by side in integrally joined enclosures (30), which detector pixels face one another along the edges, are signal-communicatively connected to one another via the, or an additional, contact redistribution layer (34) of the enclosures (30).

8. Method for producing a photon detector (3), in particular an X-ray radiation detector (3), comprising a sensor element (10) and a detector circuit (20) situated after the sensor element (10) in the direction of incident radiation, comprising the steps:
- providing (S1) the sensor element (10), wherein the sensor element (10) is formed by a semiconductor material and is sensitive to incident radiation, and wherein the sensor element (10) forms a pixel array comprising a number of sensor pixels (12),
- providing (S2) the detector circuit (20), wherein the detector circuit (20) is used to detect charge carriers generated in the semiconductor material of the sensor element (10) as a result of radiation, and comprises an integrated circuit (22) comprising a number of detector pixels (24), wherein a surface dimension of the integrated circuit (22) is smaller than the surface dimension of the sensor element (10) by more than one pixel width of the sensor pixels (12), and
- placing (S3) the detector pixels (24) in signal communication contact with the sensor pixels (12) by means of a contact redistribution layer (34) arranged on an enclosure (30), in which the integrated circuit (22) is embedded, wherein the contact redistribution layer (34) expands, in a two-dimensional manner, a contact area of the integrated circuit (22), which faces the sensor element (10) and carries contact pads assigned to each detector pixel (24), on a mating contact area of the sensor element (10) which faces the integrated circuit (22), and wherein the contact redistribution layer (34) is formed by a thin film application process,
**characterised in that**
in a further method step, which takes place in parallel with the providing (S1) of the at least one sensor element or earlier or later, a plurality of integrated circuits (22) provided for the sensor element (10) are grouped together in a shared enclosure (30) formed integrally
and, in the method step of placing in signal communication contact (S3), the integrated circuits (22) grouped together by the shared enclosure (30) are mounted on the sensor element (10), and the sensor pixels (12) that correspond to one another are placed in contact with the detector pixels of the respective integrated circuits (22),
and wherein two integrated circuits (22), which are arranged side by side in the shared enclosure (30), are placed in signal communication contact with one another using the, or an additional, contact redistribution layer (34) of the shared enclosure (30).

9. Method for producing a photon detector (3), in particular an X-ray radiation detector (3), comprising a plurality of sensor elements (10) and in each case one detector circuit (20) situated after the sensor elements (10) in the direction of incident radiation, comprising the steps:
- providing (S1) the plurality of sensor elements (10), wherein the sensor elements (10) are each formed by a semiconductor material and are sensitive to incident radiation, and wherein the sensor elements (10) each form a pixel array comprising a number of sensor pixels (12), wherein the plurality of sensor elements (10) are arranged side by side and span a sensor area of the photon detector (3),
- providing (S2) the detector circuits (20),
wherein the detector circuits (20) are used to detect charge carriers generated in the semiconductor material of the respective sensor element (10) as a result of radiation, and each comprise an integrated circuit (22) comprising a number of detector pixels (24),
wherein a surface dimension of the associated integrated circuit (22) is smaller than the surface dimension of the associated sensor element (10) by more than one pixel width of the sensor pixels (12), and
- placing (S3) the detector pixels (24) in signal communication contact with the sensor pixels (12) by means of a contact redistribution layer (34) arranged on an enclosure (30), in which the respective integrated circuit (22) is embedded, wherein the contact redistribution layer (34) expands, in a two-dimensional manner, a contact area of the integrated circuit (22), which faces the associated sensor element (10) and carries contact pads assigned to each detector pixel (24), on a mating contact area of the associated sensor element (10) which faces the integrated circuit (22), and wherein the contact redistribution layer (34) is formed by a thin film application process,
wherein more integrated circuits (22) than necessary for a single sensor element (10) are grouped together in a shared enclosure (30) formed integrally to form a detector circuit array and, in the method step of placing in signal communication contact (S3), a plurality of individual sensor elements (10) are placed on said detector circuit array,
**characterised in that**
two integrated circuits (22), which are arranged side by side in the shared enclosure (30), are placed in signal communication contact with one another using the, or an additional, contact redistribution layer (34) of the shared enclosure (30).

10. Method according to claim 8 or 9,
wherein a respective detector circuit (20) comprising the integrated circuit (22) is fabricated by fan-out wafer-level chip-scale packaging.

11. Medical X-ray apparatus (1) comprising a photon detector, in particular an X-ray radiation detector (3) according to one of claims 1 to 7.

12. Medical X-ray apparatus (1) according to claim 11, wherein the X-ray apparatus (1) is a computed tomography apparatus.

## Revendications

1. Détecteur (3) de photons, en particulier détecteur (3) du rayonnement X,
- comprenant un élément (10) capteur, qui est formé par un matériau semiconducteur, qui est sensible à du rayonnement incident et qui forme une matrice de pixel ayant un nombre de pixels (12) de capteur,
- comprenant un circuit (20) de détecteur, qui est monté en aval de l'élément (10) capteur dans le sens d'incidence du rayonnement, et qui sert à la détection de porteurs de charge créés, en raison du rayonnement, dans le matériau semiconducteur de l'élément (10) capteur, dans lequel le circuit (20) de détecteur comprend un circuit (22) intégré de commutation ayant un nombre de pixels (24) de détecteur, qui sont contactés en technique de transmission du signal par les pixels (12) de capteur,
dans lequel une étendue en surface du circuit (22) intégré de commutation est inférieure à l'étendue en surface de l'élément (10) capteur de plus d'une largeur de pixel des pixels (12) de capteur, dans lequel le circuit (20) du détecteur a un boîtier (30), qui entoure le circuit (22) intégré de commutation, dans lequel le circuit (22) intégré de commutation est incorporé et sur lequel est constituée, sur un côté (32) de pixel tourné vers l'élément (10) capteur, une couche (34) de mise en contact, dans laquelle il est constitué des points de contact pour la connexion, en technique de transmission du signal, des pixels (24) du détecteur avec les pixels (12) du capteur associés d'une manière correspondante, ainsi que des pistes (36, 38) conductrices de connexion des points de contact avec le pixel (24) du détecteur du circuit (22) intégré de commutation, dans lequel la couche (34) de mise en contact a une surface de contact du circuit (22) intégré de commutation, tournée vers l'élément (10) capteur et portant des points de contact associés à chaque pixel (24) du détecteur, appliquée par une surface à une surface de contact antagoniste de l'élément (10) capteur tournée vers le circuit (22) intégré de commutation, et dans lequel la couche (34) de mise en contact est formée par un procédé d'application en couche mince, et dans lequel à l'élément (10) capteur sont associés plusieurs circuits (20) de détecteur comprenant chacun un circuit (22) intégré de commutation, qui sont disposés à la manière d'un réseau sur la surface de contact antagoniste de l'élément (10) capteur et y sont mis en contact, de manière à ce que chaque circuit (22) intégré de commutation soit en contact avec respectivement un groupe, affecté de manière distincte, de pixels (12) de capteur, et dans lequel les boîtiers (30) d'au moins deux circuits (20) de détecteur comprenant chacun un circuit (22) intégré de commutation sont constitués entre eux d'une seule pièce en étant, en particulier monolithique, **caractérisé en ce que**
deux circuits (22) intégrés de commutation, qui sont disposés l'un à côté de l'autre par des boîtiers (30) assemblés en une pièce, sont, en utilisant la ou une autre couche (34) de mise en contact des boîtiers (30), connectés entre eux en technique de transmission du signal.

2. Détecteur (3) de photons, en particulier détecteur (3) du rayonnement X,
- comprenant plusieurs éléments (10) capteur, qui sont formés de matériau semiconducteur, qui sont sensibles à du rayonnement incident et qui forment respectivement une matrice de pixel ayant un nombre de pixels (12) de capteur, dans lequel les plusieurs éléments (10) de capteur sont disposés les uns à côté des autres et s'étendent sur une surface de capteur du détecteur (3) de photons,
- comprenant plusieurs circuits de détecteur, qui sont affectés de manière correspondante aux éléments (10) capteur, qui sont montés respectivement en aval dans le sens d'incidence du rayonnement des éléments (10) capteur et qui servent à la détection de porteurs de charge créés, en raison du rayonnement, dans le matériau semiconducteur des éléments (10) capteur, dans lequel les circuits (20) de détecteur comprennent chacun un circuit (22) intégré de commutation ayant un nombre de pixels (24) de détecteur, qui sont mis en contact en technique de transmission du signal avec les pixels (12) de capteur,
dans lequel une étendue en surface du circuit (22) intégré de commutation respectif est inférieure à l'étendue en surface de l'élément (10) capteur respectif de plus d'une largeur de pixel des pixels (12) de capteur,
dans lequel les circuits (20) de détecteur ont chacun un boîtier (30), qui entoure le circuit (22) intégré de commutation, dans lequel le circuit (22) intégré de commutation est incorporé et sur lequel est constituée sur un côté (32) de pixel, tourné vers l'élément (10) capteur, une couche (34) de mise en contact, dans laquelle il est constitué des points de contact pour la connexion, en technique de transmission du signal, des pixels (24) du détecteur avec les pixels (12) du capteur affectés d'une manière correspondante, ainsi que des pistes (36, 38) conductrices de connexion des points de contact avec les pixels (24) du détecteur du circuit (22) intégré de commutation,
dans lequel la couche (34) de mise en contact a une surface de contact du circuit (22) intégré de commutation, qui est tournée vers l'élément (10) capteur respectif, qui porte des points de contact affectés à chaque pixel (24) de détecteur et qui est appliquée par une surface à une surface de contact antagoniste de l'élément (10) capteur respectif tournée vers le circuit (22) intégré de commutation, et dans lequel la couche (34) de mise en contact est constituée par un procédé d'application en couche mince,
et dans lequel les boîtiers (30) d'au moins deux circuits (20) de détecteur, comprenant chacun un circuit (22) intégré de commutation, sont constitués entre eux d'une seule pièce, en particulier en étant monolithiques,
**caractérisé en ce que**
deux circuits (22) intégrés de commutation, qui sont disposés l'un à côté de l'autre par des boîtiers (30) assemblés en une seule pièce, sont connectés entre eux en technique de transmission du signal, en utilisant la ou une autre couche (34) de mise en contact des boîtiers (30).

3. Détecteur (3) de photons suivant la revendication 1 ou 2, dans lequel un circuit (22) intégré de commutation respectif est incorporé en étant à affleurement de surface et sans fente sur ses bords dans le boîtier (30).

4. Détecteur (3) de photons suivant l'une des revendications 1 à 3,
dans lequel un circuit (20) respectif de détecteur est fabriqué par le circuit (22) intégré de commutation au moyen d'un Fan-Out Wafer-Level Chip-Scale Packaging.

5. Détecteur (3) de photons suivant l'une des revendications 1 à 4,
dans lequel un circuit (20) respectif de détecteur a, dans la partie du boîtier (30), au moins une traversée (40) de liaison technique de transmission du signal à une unité montée en aval de commande et d'évaluation et/ou à un moyen (42) de conduite du signal monté en aval.

6. Détecteur (3) de photons suivant l'une des revendications 1 à 5,
dans lequel un circuit (20) respectif de détecteur est agencé pour l'identification d'un évènement de Charge-Sharing, par le fait que, respectivement, des pixels (24) voisins de détecteur du circuit (22) intégré de commutation respectif sont connectés entre eux en technique de transmission du signal.

7. Détecteur (3) de photons suivant l'une des revendications 1 à 6,
dans lequel pour l'identification d'un évènement de Charge-Sharing, des pixels (24) de détecteur, tournés les uns vers les autres du côté du bord, de deux circuits (22) intégrés de commutation, qui sont disposés l'un à côté de l'autre en des boîtiers (30) assemblés d'une seule pièce, sont connectés entre eux en technique de transmission du signal par la ou par une autre couche (34) de mise en contact des boîtiers (30).

8. Procédé de fabrication d'un détecteur (3) de photons, en particulier d'un détecteur (3) du rayonnement X, comportant un élément (10) capteur et un circuit (20) de détecteur monté en aval dans le sens d'incidence du rayonnement de l'élément (10) capteur, comprenant les stades
- on se procure (S1) l'élément (10) capteur, dans lequel l'élément (10) capteur est formé par un matériau semiconducteur et est sensible à du rayonnement incident, et dans lequel l'élément (10) capteur forme une matrice de pixel ayant un nombre de pixels (12) de capteur,
- on se procure (S2) le circuit (20) de détecteur, dans lequel le circuit (20) de détecteur sert à la détection de porteurs de charge créés, en raison du rayonnement, dans le matériau semiconducteur de l'élément (10) capteur et a un circuit (22) intégré de commutation ayant un nombre de pixel (24) de détecteur, dans lequel une étendue en surface du circuit (22) intégré de commutation est inférieure à l'étendue en surface de l'élément (10) capteur de plus d'une largeur de pixel des pixels (12) de capteur, et
- on met en contact (S3) en technique de transmission du signal les pixels (24) du détecteur avec les pixels (12) du capteur, au moyen d'une couche (34) de mise en contact, disposée sur un boîtier (30), dans lequel est incorporé le circuit (22) intégré de commutation, dans lequel la couche (34) de mise en contact a une surface de contact du circuit (22) intégré de commutation, qui est tournée vers l'élément (10) capteur, qui porte des points de contact affectés à chaque pixel (24) de détecteur et qui est appliquée par une surface à une surface de contact antagoniste de l'élément (10) capteur tournée vers le circuit (22) intégré de commutation, et dans lequel la couche (34) de mise en contact est formée par un procédé d'application en couche mince,
**caractérisé en ce que**
dans un autre stade du procédé, qui s'effectue parallèlement à la mise (S1) à disposition du au moins un élément capteur ou avant ou après dans le temps, on réunit plusieurs circuits (22) intégrés de commutation prévus pour l'élément (10) capteur en un seul boîtier (30) commun d'une seule pièce et, dans le stade de procédé de la mise en contact (S3) en technique de transmission du signal, on met sur l'élément (10) capteur les circuits (22) intégrés de commutation rassemblés par le boîtier (30) commun et on met les pixels (12) de capteur se correspondant l'un à l'autre en contact avec les pixels de détecteur des circuits (22) intégrés de commutation respectifs, et dans lequel deux circuits (22) intégrés de commutation, qui sont disposés l'un à côté de l'autre dans le boîtier (30) commun sont, en utilisant la ou une autre couche (34) de mise en contact du boîtier (30) commun, connectés entre eux en technique de transmission du signal.

9. Procédé de fabrication d'un détecteur (3) de photons, en particulier d'un détecteur (3) du rayonnement X, comportant plusieurs éléments (10) capteur et respectivement un circuit (20) de détecteur, monté en aval dans le sens d'incidence du rayonnement des éléments (10) capteur, comprenant les stades
- on se procure (S1) les plusieurs éléments (10) de capteur, dans lequel les éléments (10) de capteur sont formés respectivement par un matériau semiconducteur et sont sensibles à du rayonnement incident, et dans lequel les éléments (10) capteur forment respectivement une matrice de pixel ayant un nombre de pixels (12) de capteur,
dans lequel les plusieurs éléments (10) capteur sont disposés les uns à côté des autres et s'étendent sur une surface de capteur du détecteur (3) de photons,
- on se procure (S2) les circuits (20) de détecteur, dans lequel les circuits (20) de détecteur servent à la détection de porteurs de charge créés, en raison du rayonnement, dans le matériau semiconducteur de l'élément (10) capteur respectif et ont chacun un circuit (22) intégré de commutation ayant un nombre de pixels (24) de détecteur,
dans lequel une étendue en surface du circuit (22) intégré de commutation respectif est inférieure à l'étendue en surface de l'élément (10) capteur respectif de plus d'une largeur de pixel des pixels (12) de capteur, et
- on met en contact (S3) en technique de transmission du signal les pixels (24) de détecteur avec les pixels (12) de capteur au moyen d'une couche (34) de mise en contact montée sur un boîtier (30), dans lequel le circuit (22) intégré de commutation respectif est incorporé, dans lequel la couche (34) de mise en contact a une surface de contact du circuit (22) intégré de commutation, qui est tournée vers l'élément (10) capteur respectif, qui porte des points de contact affectés à chaque pixel (24) de détecteur et qui est appliquée par une surface à une surface de contact antagoniste de l'élément (10) capteur respectif, tournée vers le circuit (22) intégré de commutation, et dans lequel la couche (34) de mise en contact est constituée par un procédé d'application en couche mince,
dans lequel on rassemble plusieurs circuits (22) intégrés de commutation, comme nécessaires pour un élément (10) capteur individuel, dans un boîtier (30) commun constitué d'une seule pièce en un réseau de circuits de détecteur et dans le stade du procédé de la mise (S3) en contact en technique de transmission du signal, on applique plusieurs éléments (10) capteur à ce réseau de circuits de détecteur,
**caractérisé en ce que**
deux circuits (22) intégrés de commutation, qui sont disposés l'un à côté de l'autre dans le boîtier (30) commun sont, en utilisant la ou une autre couche (34) de mise en contact du boîtier (30) commun, connectés entre eux en technique de transmission du signal.

10. Procédé suivant la revendication 8 ou 9,
dans lequel on fabrique un circuit (20) respectif de détecteur ayant le circuit (22) intégré de commutation au moyen d'un Fan-Out Wafer-Level Chip-Scale Packaging.

11. Appareil (1) médical à rayons X, comprenant un détecteur de photons, en particulier un détecteur (3) du rayonnement X suivant l'une des revendications 1 à 7.

12. Appareil (1) médical à rayons X suivant la revendication 11, dans lequel l'appareil (1) à rayons X est un tomodensitomètre assisté par ordinateur.
